(19) Europäisches Patentamt
European Patent Office
Office européen des brevets

(11) EP 4 286 428 A1

(12) **EUROPEAN PATENT APPLICATION**
published in accordance with Art. 153(4) EPC

(43) Date of publication:
**06.12.2023 Bulletin 2023/49**

(21) Application number: **22745727.2**

(22) Date of filing: **21.01.2022**

(51) International Patent Classification (IPC):
***C08F 120/34*** *(2006.01)* ***C09K 3/00*** *(2006.01)*
***C12Q 1/6844*** *(2018.01)* ***C12Q 1/686*** *(2018.01)*
***C12Q 1/6888*** *(2018.01)*

(52) Cooperative Patent Classification (CPC):
**C08F 120/34; C09K 3/00; C12Q 1/6844; C12Q 1/686; C12Q 1/6888**

(86) International application number:
**PCT/JP2022/002102**

(87) International publication number:
**WO 2022/163506 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.01.2021 JP 2021012400**

(71) Applicant: **NOF Corporation**
**Shibuya-ku**
**Tokyo 150-6019 (JP)**

(72) Inventors:
- **SUZUKI, Hirotaka**
  **Kawasaki-shi, Kanagawa 210-0865 (JP)**
- **KONDO, Seina**
  **Kawasaki-shi, Kanagawa 210-0865 (JP)**
- **MATSUDA, Masaru**
  **Kawasaki-shi, Kanagawa 210-0865 (JP)**

(74) Representative: **Heubeck, Christian**
**Weickmann & Weickmann**
**Patent- und Rechtsanwälte PartmbB**
**Postfach 860 820**
**81635 München (DE)**

(54) **SENSITIZER FOR NUCLEIC ACID AMPLIFICATION, COMPOSITION FOR NUCLEIC ACID AMPLIFICATION, AND TEST KIT**

(57) The present invention provides a sensitizer for nucleic acid amplification which is a polymer containing a constitutional unit derived from a monomer represented by the following formula (1):

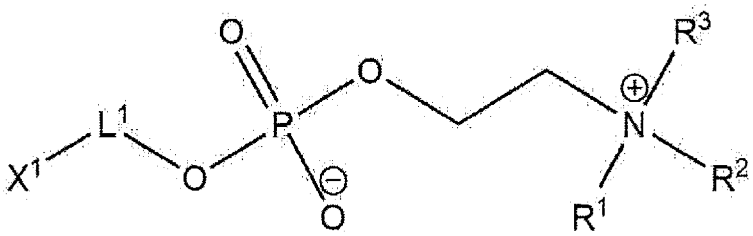

(1)

wherein symbols are as defined in the specification.

EP 4 286 428 A1

## Description

[Technical Field]

The present invention relates to sensitizers for nucleic acid amplification, compositions for nucleic acid amplification, and test kits.

[Background Art]

A nucleic acid amplification method is a method for amplifying the target nucleic acid from several copies to tens of thousands of times more copies, and is actively used in various areas for genetic testing, microbial testing, and viral testing.

A representative method of nucleic acid amplification method is the Polymerase Chain Reaction (PCR) method. In a typical PCR method, nucleic acid is amplified by repeating the three steps of (1) denaturation of template DNA (separation from double-stranded DNA to single-stranded DNA), (2) annealing of primer to single-stranded template DNA, and (3) elongation of primer by DNA polymerase. In a classical end-point PCR method, an amplification product is detected by visualizing same with a fluorescent compound or the like or by measuring the turbidity of the solution at the reaction end point after performing a predetermined reaction cycle.

A typical PCR method is a method for amplifying DNA. It can also be applied to the amplification of RNA, and such PCR method is called a reverse transcription polymerase chain reaction method (hereinafter sometimes to be abbreviated as "RT-PCR method"). In the RT-PCR method, complementary DNA (hereinafter sometimes to be abbreviated as "cDNA") is synthesized from RNA by an enzymatic reaction using reverse transcriptase, and PCR is performed using this cDNA as a template to amplify RNA.

A method for determining the amount of initial nucleic acid based on the amount of amplified product obtained by the PCR method is also known, and such PCR method is called a quantitative polymerase chain reaction method (hereinafter sometimes to be abbreviated as "qPCR method"). In a narrow sense, the qPCR method is a real-time PCR method, which is a method of visualizing the amount of DNA amplified in each cycle of PCR, by using a fluorescent DNA staining reagent or a fluorescent probe. In particular, a method using a fluorescent probe is known as a highly reliable method because it can specifically detect amplification of the target nucleic acid.

A reverse transcription-quantitative PCR method (hereinafter sometimes to be abbreviated as "RT-qPCR method"), which is a combination of the RT-PCR method and the qPCR method, is also known. The RT-qPCR method is further divided into a 2-step method in which cDNA synthesis and qPCR method are performed in separate containers, and a 1-step method in which these are performed as a series of reactions in the same container. Of these, the 1-step method is superior in terms of ease of operation, little contamination from outside the system, and high detection sensitivity for nucleic acid as a measurement target (hereinafter sometimes to be abbreviated as "sensitivity").

In recent years, moreover, a method called a digital PCR method (hereinafter sometimes to be abbreviated as "dPCR method") has also been developed in which a reaction liquid is dispensed to some tens to tens of thousands of extremely small compartments and PCR is performed all at once by applying microchannel formation technology and the like, and the concentration of initial nucleic acid is determined using a statistical model from the proportion of the compartments with amplification. The dPCR method is an advanced end-point PCR method.

In addition to the purpose of determining the presence or absence and/or the amount of the target nucleic acid as described above, the PCR method is also used for the purpose of determining the base sequence of the target nucleic acid (sequencing). The sequencing PCR method includes various methods represented by the Sanger method, and the basic principle thereof is the end-point PCR method.

When using nucleic acid amplification methods, sensitivity is often a problem in all of qualification, quantification, and sequence determination. Therefore, various techniques for promoting PCR have been investigated.

For example, it is widely practiced to increase sensitivity by adding salts (e.g., potassium chloride, ammonium sulfate, etc.), betaine, polyhydric alcohols, and the like to a reaction liquid in the PCR method. However, there is a limit to the improvement in sensitivity with the addition of these components, and sensitivity improvement to a higher level has been demanded.

For example, a method of destabilizing double-stranded DNA by adding a single-stranded DNA binding protein (SSB) is known. Patent Literature 1 discloses a mutant PCNA (proliferating nuclear antigen) monomer as a highly versatile DNA replication promoter (additive) for promoting the elongation reaction of DNA. However, these additives composed of proteins have problems such as difficulty in large-scale production, problems in storage stability, and high production costs.

[Citation List]

[Patent Literature]

[PTL 1]
WO 2007/004654

[Summary of Invention]

[Technical Problem]

The present invention aims to provide a sensitizer for nucleic acid amplification which is superior in mass productivity and preservation stability as compared with additives composed of proteins.

[Solution to Problem]

The present inventors have conducted intensive studies and found that a polymer containing a constitutional unit derived from a monomer represented by the following formula (1) :

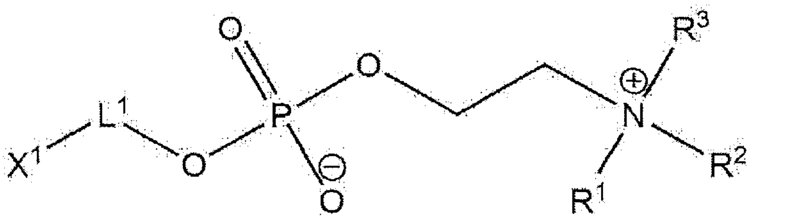

(1)

can be preferably used as a sensitizer for nucleic acid amplification. Based on this finding, the present invention provides the following.
[1] A sensitizer for nucleic acid amplification which is a polymer comprising a constitutional unit derived from a monomer represented by the formula (1):

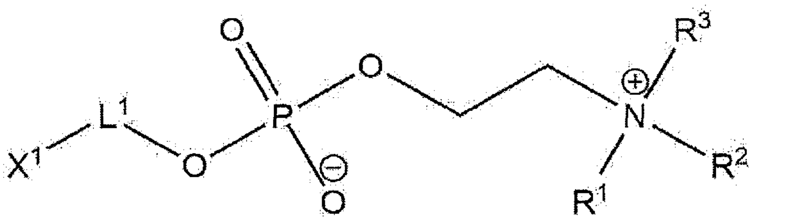

(1)

wherein

$X^1$ is a (meth)acryloyloxy group or a (meth)acryloylamino group,
$L^1$ is an alkylene group having 2 to 4 carbon atoms and optionally having one hydroxy group, or an alkyleneoxyalkylene group having 2 to 4 carbon atoms, and
$R^1$ to $R^3$ are each independently an alkyl group having 1 to 3 carbon atoms.

[2] The sensitizer of the aforementioned [1], wherein the sensitizer is a homopolymer comprising one kind of constitutional unit derived from the aforementioned monomer represented by the formula (1).
[3] The sensitizer of the aforementioned [1], wherein the sensitizer is a copolymer further comprising a constitutional unit derived from a monomer represented by the formula (2):

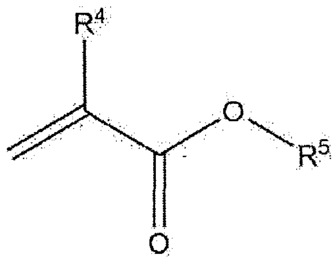

(2)

wherein

$R^4$ is a hydrogen atom or a methyl group, and
$R^5$ is a hydrogen atom or an alkyl group having 1 to 20 carbon atoms.

[4] The sensitizer of the aforementioned [3], wherein $R^5$ is an alkyl group having 12 to 18 carbon atoms.
[5] The sensitizer of the aforementioned [1], [3], or [4], wherein the sensitizer is a copolymer further comprising a constitutional unit derived from a monomer represented by the formula (3):

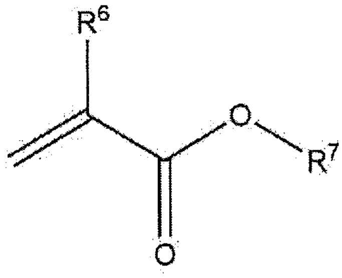

(3)

wherein

$R^6$ is a hydrogen atom or a methyl group, and
$R^7$ is an alkyl group having 3 to 6 carbon atoms, and two or more hydroxy groups.

[6] A composition for nucleic acid amplification, comprising the sensitizer of any one of the aforementioned [1] to [5].
[7] The composition of the aforementioned [6], wherein the composition is used for a reverse transcription polymerase chain reaction method.
[8] The composition of the aforementioned [6] or [7], wherein the composition is used for a quantitative polymerase chain reaction method.
[9] The composition of any one of the aforementioned [6] to [8], further comprising a primer.
[10] The composition of the aforementioned [9], wherein the primer is an oligonucleotide having a length of 10 to 40 bases.
[11] The composition of the aforementioned [9] or [10], wherein the primer has a concentration of 0.1 to 3.0 $\mu$M.
[12] A test kit comprising the composition of any one of the aforementioned [6] to [11].
[13] The test kit of the aforementioned [12], wherein the test kit is for a clinical test.
[14] The test kit of the aforementioned [12] or [13], wherein an object of the test is a virus.
[15] A method for amplifying a nucleic acid, comprising using the polymer of any one of the aforementioned [1] to [5] as a sensitizer.
[16] A method for amplifying a nucleic acid, comprising preparing a reaction liquid for nucleic acid amplification by mixing the composition of any one of the aforementioned [6] to [11] and a sample comprising a nucleic acid to be amplified.
[17] The method of the aforementioned [15] or [16] which is a reverse transcription polymerase chain reaction method.
[18] The method of any one of the aforementioned [15] to [17] which is a quantitative polymerase chain reaction method.

[Advantageous Effects of Invention]

The sensitizer for nucleic acid amplification of the present invention can improve detection sensitivity of nucleic acids in the nucleic acid amplification methods. In addition, since the sensitizer for nucleic acid amplification of the present invention is a synthetic polymer, it is superior in the mass productivity and preservation stability, as compared with additives composed of proteins.

[Description of Embodiments]

The present invention is described in detail below.

In the present specification, the "(meth)acryloyloxy group" basically means an "acryloyloxy group or methacryloyloxy group". When a plurality of the (meth)acryloyloxy groups may be present, the "(meth)acryloyloxy group" means an "acryloyloxy group and/or methacryloyloxy group". Other terms similar to the "(meth)acryloyloxy group" also mean the same as the "(meth)acryloyloxy group".

When stepwise numerical ranges are described in the present specification, the lower limit and the upper limit of each numerical range can be combined. In the case of, for example, "preferably 10 to 100, more preferably 20 to 90", the preferred lower limit 10 can be combined with the more preferred upper limit 90 (i.e., the numerical range of "10 to 90" is also within the range of the present specification).

[Sensitizer for nucleic acid amplification]

The sensitizer for nucleic acid amplification of the present invention (hereinafter sometimes to be indicated as "the sensitizer of the present invention") is a polymer (hereinafter sometimes to be abbreviated as "the polymer of the present invention") containing a constitutional unit derived from a monomer (hereinafter sometimes to be abbreviated as "monomer (1)") represented by the following formula (1):

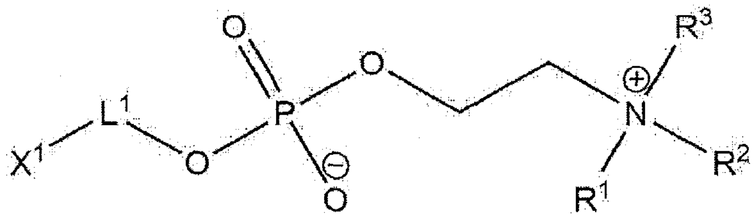

(1)

As used herein, the sensitizer for nucleic acid amplification means an additive for improving the detection sensitivity of nucleic acids in the nucleic acid amplification method.

Examples of the nucleic acid amplification method include Polymerase Chain Reaction (PCR) method, Loop mediated isothermal Amplification (LAMP) method, Transcription Mediated Amplification (TMA) method, Isothermal and Chimeric primer-initiated Amplification of Nucleic acids (IICAN) method, Strand Displacement Amplification (SDA) method, Ligase Chain Reaction (LCR) method, Nucleic Acid Sequence-Based Amplification (NASBA) method, and the like. The nucleic acid amplification method is preferably a PCR method. That is, the sensitizer of the present invention is preferably used in the polymerase chain reaction method.

As the PCR method, the aforementioned reverse transcription polymerase chain reaction method is preferred. That is, the sensitizer of the present invention is preferably used in the reverse transcription polymerase chain reaction method.

As the PCR method, the aforementioned quantitative polymerase chain reaction method is preferred. That is, the nucleus sensitizer of the present invention is preferably used in the quantitative polymerase chain reaction method.

Only one kind of the sensitizer of the present invention may be used, or two or more kinds thereof may be used in combination. In addition, the sensitizer of the present invention may be used in combination with other additives.

The constitutional unit derived from monomer (1) (hereinafter sometimes to be abbreviated as "constitutional unit (1)") means a constitutional unit having a structure formed by the reaction of a carbon-carbon double bond of the (meth)acryloyl group contained in monomer (1). The constitutional units derived from other monomers means the same as the constitutional units derived from monomer (1).

Only one kind of monomer (1) may be used, or two or more kinds thereof may be used in combination. That is, the sensitizer of the present invention may be a homopolymer consisting of one kind of constitutional unit (1), or a copolymer containing two or more kinds of constitutional units (1). The aforementioned copolymer may be a random copolymer, a block copolymer, or a copolymer containing both a random portion and a block portion. When the polymer

of the present invention does not contain constitutional units other than the constitutional unit (1), the sensitizer of the present invention is preferably a homopolymer consisting of one kind of constitutional unit (1), more preferably a homopolymer consisting of a constitutional unit derived from 2-(meth)acryloyloxyethyl phosphorylcholine, further preferably a homopolymer consisting of a constitutional unit derived from 2-methacryloyloxyethyl phosphorylcholine.

The groups in the formula (1) are described in order below. In the formula (1), $X^1$ is a (meth)acryloyloxy group (i.e., $CH_2$=CR-CO-O-, R: hydrogen atom or a methyl group) or a (meth)acryloylamino group (i.e., $CH_2$=CR-CO-NH-, R: hydrogen atom or a methyl group). From the aspect of the availability of the starting materials, $X^1$ is preferably a (meth)acryloyloxy group, more preferably a methacryloyloxy group.

In the formula (1), $L^1$ is an alkylene group having 2 to 4 carbon atoms and optionally having one hydroxy group, or an alkyleneoxyalkylene group having 2 to 4 carbon atoms. The aforementioned alkylene group may be linear or branched chain. Examples of the alkylene group having 2 to 4 carbon atoms and optionally having one hydroxy group include $-C_2H_4-$. Examples of the alkyleneoxyalkylene group having 2 to 4 carbon atoms include $-C_2H_4-O-C_2H_4-$. From the aspect of the availability of the starting materials, $L^1$ is preferably $-C_2H_4-$ or $-C_2H_4-O-C_2H_4-$, more preferably $-C_2H_4-$ (i.e., ethylene group).

In the formula (1), $R^1$ to $R^3$ are each independently an alkyl group having 1 to 3 carbon atoms. The aforementioned alkyl group may be linear or branched chain. Examples of the alkyl group having 1 to 3 carbon atoms include methyl group, ethyl group, propyl group, and the like. From the aspect of the availability of the starting materials, $R^1$ to $R^3$ are preferably methyl groups.

Preferred monomer (1) is a monomer in which $X^1$ is a (meth)acryloyloxy group, $L^1$ is $-C_2H_4-$ or $-C_2H_4-O-C_2H_4-$, and $R^1$ to $R^3$ are methyl groups. More preferred monomer (1) is a monomer in which $X^1$ is a (meth)acryloyloxy group, $L^1$ is an ethylene group, and $R^1$ to $R^3$ are methyl groups (i.e., 2-(meth)acryloyloxyethyl phosphorylcholine). Further preferred monomer is 2-methacryloyloxyethyl phosphorylcholine. A commercially available product can be used for monomer (1).

The polymer of the present invention may further contain, in addition to constitutional unit (1), a constitutional unit (hereinafter sometimes to be abbreviated as "constitutional unit (2)") derived from a monomer (hereinafter sometimes to be abbreviated as "monomer (2)") represented by the formula (2):

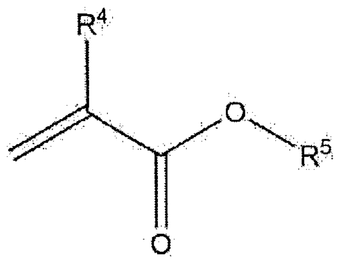

(2)

Only one kind of monomer (2) may be used, or two or more kinds thereof may be used in combination. That is, the polymer of the present invention may be a copolymer containing one or more kinds of constitutional unit (1) and one or more kinds of constitutional unit (2). The aforementioned copolymer may be a random copolymer, a block copolymer, or a copolymer containing both a random portion and a block portion.

The groups in the formula (2) are described in order below. In the formula (2), $R^4$ is a hydrogen atom or a methyl group. From the aspect of the preservation stability of the polymer, $R^4$ is preferably a methyl group.

In the formula (2), $R^5$ is a hydrogen atom or an alkyl group having 1 to 20 carbon atoms. The aforementioned alkyl group may be linear or branched chain. Examples of the alkyl group having 1 to 20 carbon atoms include methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, and the like.

From the aspect of sensitizing effect, $R^5$ is preferably an alkyl group having 1 to 20 carbon atoms, more preferably an alkyl group having 2 to 20 carbon atoms, further preferably an alkyl group having 12 to 18 carbon atoms, particularly preferably a linear alkyl group having 12 to 18 carbon atoms. When the polymer of the present invention further contains a constitutional unit derived from the monomer represented by the below-mentioned formula (3), $R^5$ is preferably an alkyl group having 3 to 6 carbon atoms.

Specific examples of monomer (2) include (meth)acrylic acid, methyl (meth)acrylate, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, stearyl (meth)acrylate, and the like. A commercially available product can be used for monomer (2).

In the aforementioned specific examples of monomer (2), (meth)acrylic acid, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, and stearyl (meth)acrylate is preferable, butyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, and stearyl (meth)acrylate are more preferred, butyl (meth)acrylate and stearyl (meth)acrylate are further preferred, and butyl methacrylate and stearyl methacrylate are particularly preferred.

When the polymer of the present invention contains constitutional unit (1) and constitutional unit (2), from the aspect of sensitizing effect, the amount of constitutional unit (1) (i.e., monomer (1) used in polymerization) is preferably 30 to 99 mol, more preferably 30 to 90 mol, further preferably 50 to 90 mol, particularly preferably 75 to 90 mol, and the amount of constitutional unit (2) (i.e., monomer (2) used in polymerization) is preferably 1 to 70 mol, more preferably 10 to 70 mol, further preferably 10 to 50 mol, particularly preferably 10 to 25 mol, each with respect to the total 100 mol of the constitutional unit (1) and constitutional unit (2) (i.e., total 100 mol of monomer (1) and monomer (2) used in polymerization).

The polymer of the present invention may further contain, in addition to constitutional unit (1), a constitutional unit (hereinafter sometimes to be abbreviated as "constitutional unit (3)") derived from a monomer (hereinafter sometimes to be abbreviated as "monomer (3)") represented by the formula (3):

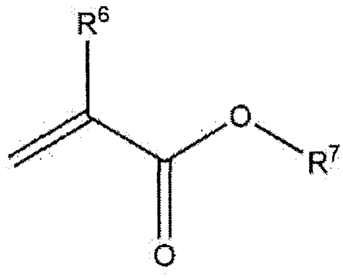

(3)

Only one kind of monomer (3) may be used, or two or more kinds thereof may be used in combination. That is, the polymer of the present invention may be a copolymer containing one or more kinds of constitutional unit (1) and one or more kinds of constitutional unit (3), or a copolymer containing one or more kinds of constitutional unit (1), one or more kinds of constitutional unit (2), and one or more kinds of constitutional unit (3). The aforementioned copolymer may be a random copolymer, a block copolymer, or a copolymer containing both a random portion and a block portion.

The groups in the formula (3) are described in order below. $R^6$ is a hydrogen atom or a methyl group. From the aspect of the preservation stability of the polymer, $R^6$ is preferably a methyl group.

In the formula (3), $R^7$ is an alkyl group having 3 to 6 carbon atoms, and two or more hydroxy groups. The number of hydroxy groups in $R^7$ is preferably 2 to 5. The aforementioned alkyl group may be linear or branched chain. Examples of the alkyl group having 3 to 6 carbon atoms include propyl group, butyl group, pentyl group, hexyl group, and the like.

Specific examples of monomer (3) include, glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, sorbitol mono(meth)acrylate, and the like. Among these, glycerol mono(meth)acrylate and xylitol mono(meth)acrylate are preferred, glycerol mono(meth)acrylate is more preferred, and glycerol monomethacrylate is further preferred.

A commercially available product can be used for monomer (3), or may be produced by a known method. For example, monomer (3) can be produced by an esterification reaction of (meth)acrylic acid or a derivative thereof (e.g., acid chloride) with a polyhydric alcohol having 3 or more hydroxy groups. Eesterification reaction is well known, those of ordinary skill in the art can set the conditions appropriately and perform the reaction.

When the polymer of the present invention contains constitutional unit (1) and constitutional unit (3), from the aspect of sensitizing effect, the amount of constitutional unit (1) (i.e., monomer (1) used in polymerization) is preferably 30 to 80 mol, more preferably 30 to 70 mol, further preferably 30 to 60 mol, and the amount of constitutional unit (3) (i.e., monomer (3) used in polymerization) is preferably 20 to 70 mol, more preferably 30 to 70 mol, further preferably 40 to 70 mol, each with respect to the total 100 mol of the constitutional unit (1) and constitutional unit (3) (i.e., total 100 mol of monomer (1) and monomer (3) used in polymerization).

When the polymer of the present invention contains constitutional unit (1), constitutional unit (2), and constitutional unit (3), from the aspect of sensitizing effect, the amount of constitutional unit (1) (i.e., monomer (1) used in

polymerization) is preferably 30 to 80 mol, more preferably 30 to 70 mol, further preferably 30 to 60 mol, the amount of constitutional unit (2) (i.e., monomer (2) used in polymerization) is preferably 10 to 60 mol, more preferably 20 to 60 mol, further preferably 30 to 60 mol, and the amount of constitutional unit (3) (i.e., monomer (3) used in polymerization) is preferably 10 to 60 mol, more preferably 10 to 50 mol, further preferably 10 to 40 mol, each with respect to the total 100 mol of the constitutional unit (1), constitutional unit (2), and constitutional unit (3) (i.e., total 100 mol of monomer (1), monomer (2), and monomer (3) used in polymerization).

The polymer of the present invention may contain, as long as the effect of the present invention is not impaired, other constitutional units derived from other monomers different from the aforementioned monomers (1) to (3). Only one kind of other monomer may be used, or two or more kinds thereof may be used in combination. Other monomer is not particularly limited, and examples thereof include benzyl (meth)acrylate, isobornyl (meth)acrylate, and the like. The amount of other constitutional unit in the polymer of the present invention is preferably not more than 20 mol% with respect to the total constitutional units. More preferably, the polymer of the present invention does not contain other constitutional units.

The polymer of the present invention is preferably at least one selected from the group consisting of a homopolymer consisting of one kind of constitutional unit (1), a copolymer consisting of constitutional unit (1) and constitutional unit (2), and a copolymer consisting of constitutional unit (1), constitutional unit (2), and constitutional unit (3). More preferably, it is a homopolymer consisting of one kind of constitutional unit (1), a copolymer consisting of constitutional unit (1) and constitutional unit (2), or a copolymer consisting of constitutional unit (1), constitutional unit (2), and constitutional unit (3). In the present specification, the "homopolymer consisting of one kind of constitutional unit (1)" means a homopolymer whose all constitutional units (repeating units) consist of one kind of constitutional unit (1), the "copolymer consisting of constitutional unit (1) and constitutional unit (2)" means a copolymer whose all constitutional units (repeating units) consist of constitutional unit (1) and constitutional unit (2), and the "copolymer consisting of constitutional unit (1), constitutional unit (2), and constitutional unit (3)" means a copolymer whose all constitutional units (repeating units) consist of constitutional unit (1), constitutional unit (2), and constitutional unit (3). Other similar expressions mean the same.

While the weight average molecular weight of the polymer of the present invention is not particularly limited, it is preferably 10,000 to 1,000,000. The weight average molecular weight can be determined based on polyethylene glycol by gel filtration chromatography using, for example, EcoSEC system (manufactured by Tosoh Corporation) or the like.

The polymer of the present invention can be produced by known methods (e.g., the method described in WO 2018/216628).

The amount of the sensitizer of the present invention (that is, the polymer of the present invention) is determined by the concentration in the composition for nucleic acid amplification described below. The concentration of the sensitizer of the present invention in the aforementioned composition is preferably 0.00001 to 10 w/v%, more preferably 0.001 to 1 w/v%, further preferably 0.01 to 0.5 w/v%, from the aspects of the sensitizing effect and suppression of increase in the viscosity of the aforementioned composition. When two or more kinds of sensitizers are used, the aforementioned concentration means the total concentration of the two or more kinds of sensitizers. When two or more kinds of the following other components are used, the concentrations described for such other components also refer to the total of the concentrations of the two or more components.

[Examples of preferred sensitizer of the present invention (polymer of the present invention)]

Examples of preferred sensitizer of the present invention (polymer of the present invention) include sensitizer (I) of the present invention (polymer (I) of the present invention) to sensitizer (IV) of the present invention (polymer (IV) of the present invention) below.

<Sensitizer (I) of the present invention (polymer (I) of the present invention)>

The sensitizer (I) of the present invention (polymer (I) of the present invention) is at least one selected from the group consisting of the following homopolymer (I-1), copolymer (1-2), and copolymer (I-3), preferably the following homopolymer (I-1), copolymer (I-2), or copolymer (I-3):

homopolymer (I-1) consisting of constitutional unit (1) derived from 2-(meth)acryloyloxyethyl phosphorylcholine;
copolymer (I-2) consisting of constitutional unit (1) derived from 2-(meth)acryloyloxyethyl phosphorylcholine, and constitutional unit (2) derived from (meth)acrylic acid, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, or stearyl (meth)acrylate, in which the amount of constitutional unit (1) is 30 to 99 mol and the amount of constitutional unit (2) is 1 to 70 mol each with

respect to the total 100 mol of constitutional unit (1) and constitutional unit (2); and

copolymer (I-3) consisting of constitutional unit (1) derived from 2-(meth)acryloyloxyethyl phosphorylcholine, constitutional unit (2) derived from (meth)acrylic acid, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, or stearyl (meth)acrylate, and constitutional unit (3) derived from glycerol mono(meth)acrylate or xylitol mono(meth)acrylate, in which

the amount of constitutional unit (1) is 30 to 80 mol, the amount of constitutional unit (2) is 10 to 60 mol, and the amount of constitutional unit (3) is 10 to 60 mol each with respect to the total 100 mol of constitutional unit (1), constitutional unit (2), and constitutional unit (3).

In the sensitizer (I) of the present invention (polymer (I) of the present invention), it is preferred that the amount of constitutional unit (1) in copolymer (1-2) is 30 to 90 mol, and the amount of constitutional unit (2) therein is 10 to 70 mol, and the amount of constitutional unit (1) in copolymer (I-3) is 30 to 70 mol, the amount of constitutional unit (2) therein is 20 to 60 mol, and the amount of constitutional unit (3) therein is 10 to 50 mol. The standard for the amounts of the aforementioned constitutional units in copolymer (1-2) is a total 100 mol of constitutional unit (1) and constitutional unit (2). The standard for the amounts of the aforementioned constitutional units in copolymer (1-3) is a total 100 mol of constitutional unit (1), constitutional unit (2), and constitutional unit (3).

In the sensitizer (I) of the present invention (polymer (I) of the present invention), it is more preferred that the amount of constitutional unit (1) in copolymer (I-2) is 50 to 90 mol, and the amount of constitutional unit (2) therein is 10 to 50 mol, and the amount of constitutional unit (1) in copolymer (I-3) is 30 to 60 mol, the amount of constitutional unit (2) therein is 30 to 60 mol, and the amount of constitutional unit (3) therein is 10 to 40 mol. The standard for the amounts of the aforementioned constitutional units in copolymer (I-2) is a total 100 mol of constitutional unit (1) and constitutional unit (2). The standard for the amounts of the aforementioned constitutional units in copolymer (I-3) is a total 100 mol of constitutional unit (1), constitutional unit (2), and constitutional unit (3).

In the sensitizer (I) of the present invention (polymer (I) of the present invention), it is further preferred that the amount of constitutional unit (1) in copolymer (I-2) is 75 to 90 mol, and the amount of constitutional unit (2) therein is 10 to 25 mol, and the amount of constitutional unit (1) in copolymer (1-3) is 30 to 60 mol, the amount of constitutional unit (2) therein is 30 to 60 mol, and the amount of constitutional unit (3) therein is 10 to 40 mol. The standard for the amounts of the aforementioned constitutional units in copolymer (I-2) is a total 100 mol of constitutional unit (1) and constitutional unit (2). The standard for the amounts of the aforementioned constitutional units in copolymer (I-3) is a total 100 mol of constitutional unit (1), constitutional unit (2), and constitutional unit (3).

<Sensitizer (II) of the present invention (polymer (II) of the present invention)>

The sensitizer (II) of the present invention (polymer (II) of the present invention) is at least one selected from the group consisting of the following homopolymer (II-1), copolymer (II-2), and copolymer (II-3), preferably the following homopolymer (II-1), copolymer (II-2), or copolymer (II-3):

homopolymer (II-1) consisting of constitutional unit (1) derived from methacryloyloxyethyl phosphorylcholine;

copolymer (II-2) consisting of constitutional unit (1) derived from methacryloyloxyethyl phosphorylcholine, and constitutional unit (2) derived from butyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, or stearyl (meth)acrylate, in which

the amount of constitutional unit (1) is 30 to 90 mol and the amount of constitutional unit (2) is 10 to 70 mol each with respect to the total 100 mol of constitutional unit (1) and constitutional unit (2); and

copolymer (II-3) consisting of constitutional unit (1) derived from methacryloyloxyethyl phosphorylcholine, constitutional unit (2) derived from butyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, or stearyl (meth)acrylate, and constitutional unit (3) derived from glycerol mono(meth)acrylate, in which

the amount of constitutional unit (1) is 30 to 70 mol, the amount of constitutional unit (2) is 20 to 60 mol, and the amount of constitutional unit (3) is 10 to 50 mol each with respect to the total 100 mol of constitutional unit (1), constitutional unit (2), and constitutional unit (3).

In the sensitizer (II) of the present invention (polymer (II) of the present invention), it is preferred that the amount of constitutional unit (1) in copolymer (II-2) is 50 to 90 mol, and the amount of constitutional unit (2) therein is 10 to 50 mol, and the amount of constitutional unit (1) in copolymer (II-3) is 30 to 60 mol, the amount of constitutional unit (2) therein is 30 to 60 mol, and the amount of constitutional unit (3) therein is 10 to 40 mol. The standard for the amounts

of the aforementioned constitutional units in copolymer (II-2) is a total 100 mol of constitutional unit (1) and constitutional unit (2). The standard for the amounts of the aforementioned constitutional units in copolymer (II-3) is a total 100 mol of constitutional unit (1), constitutional unit (2), and constitutional unit (3).

In the sensitizer (II) of the present invention (polymer (II) of the present invention), it is more preferred that the amount of constitutional unit (1) in copolymer (II-2) is 75 to 90 mol, and the amount of constitutional unit (2) therein is 10 to 25 mol, and the amount of constitutional unit (1) in copolymer (II-3) is 30 to 60 mol, the amount of constitutional unit (2) therein is 30 to 60 mol, and the amount of constitutional unit (3) therein is 10 to 40 mol. The standard for the amounts of the aforementioned constitutional units in copolymer (II-2) is a total 100 mol of constitutional unit (1) and constitutional unit (2). The standard for the amounts of the aforementioned constitutional units in copolymer (II-3) is a total 100 mol of constitutional unit (1), constitutional unit (2), and constitutional unit (3).

<Sensitizer (III) of the present invention (polymer (III) of the present invention)>

The sensitizer (III) of the present invention (polymer (III) of the present invention) is at least one selected from the group consisting of the following homopolymer (III-1), copolymer (III-2), and copolymer (III-3), preferably the following homopolymer (III-1), copolymer (III-2), or copolymer (III-3):

homopolymer (III-1) consisting of constitutional unit (1) derived from methacryloyloxyethyl phosphorylcholine;
copolymer (III-2) consisting of constitutional unit (1) derived from methacryloyloxyethyl phosphorylcholine, and constitutional unit (2) derived from butyl (meth)acrylate or stearyl (meth)acrylate, in which
the amount of constitutional unit (1) is 50 to 90 mol and the amount of constitutional unit (2) is 10 to 50 mol each with respect to the total 100 mol of constitutional unit (1) and constitutional unit (2); and
copolymer (III-3) consisting of constitutional unit (1) derived from methacryloyloxyethyl phosphorylcholine, constitutional unit (2) derived from butyl (meth)acrylate or stearyl (meth)acrylate, and constitutional unit (3) derived from glycerol monomethacrylate, in which
the amount of constitutional unit (1) is 30 to 60 mol, the amount of constitutional unit (2) is 30 to 60 mol, and the amount of constitutional unit (3) is 10 to 40 mol each with respect to the total 100 mol of constitutional unit (1), constitutional unit (2), and constitutional unit (3).

In the sensitizer (III) of the present invention (polymer (III) of the present invention), it is preferred that the amount of constitutional unit (1) in copolymer (III-2) is 75 to 90 mol, and the amount of constitutional unit (2) therein is 10 to 25 mol. The standard for the amounts of the aforementioned constitutional units in copolymer (III-2) is a total 100 mol of constitutional unit (1) and constitutional unit (2).

<Sensitizer (IV) of the present invention (polymer (IV) of the present invention)>

The sensitizer (IV) of the present invention (polymer (IV) of the present invention) is at least one selected from the group consisting of the following homopolymer (IV-1), copolymer (IV-2), and copolymer (IV-3), preferably the following homopolymer (IV-1), copolymer (IV-2), or copolymer (IV-3):

homopolymer (IV-1) consisting of constitutional unit (1) derived from methacryloyloxyethyl phosphorylcholine;
copolymer (IV-2) consisting of constitutional unit (1) derived from methacryloyloxyethyl phosphorylcholine, and constitutional unit (2) derived from butyl methacrylate or stearyl methacrylate, in which
the amount of constitutional unit (1) is 75 to 90 mol and the amount of constitutional unit (2) is 10 to 25 mol each with respect to the total 100 mol of constitutional unit (1) and constitutional unit (2); and
copolymer (IV-3) consisting of constitutional unit (1) derived from methacryloyloxyethyl phosphorylcholine, constitutional unit (2) derived from butyl methacrylate or stearyl methacrylate, and constitutional unit (3) derived from glycerol monomethacrylate, in which
the amount of constitutional unit (1) is 30 to 60 mol, the amount of constitutional unit (2) is 30 to 60 mol, and the amount of constitutional unit (3) is 10 to 40 mol each with respect to the total 100 mol of constitutional unit (1), constitutional unit (2), and constitutional unit (3).

[Composition for nucleic acid amplification]

The present invention also provides a composition for nucleic acid amplification containing the sensitizer of the present invention (hereinafter sometimes to be indicated as " the composition of the present invention"). In the composition of the present invention, only one kind of the sensitizer of the present invention may be used, or two or more kinds thereof may be used in combination. As used herein, the composition for nucleic acid amplification means a composition

used in a nucleic acid amplification method. The nucleic acid amplification method is as described above. The nucleic acid amplification method is preferably a PCR method. That is, the composition of the present invention is preferably used in the polymerase chain reaction method.

As the PCR method, the aforementioned reverse transcription polymerase chain reaction method is preferred. That is, the composition of the present invention is preferably used in the reverse transcription polymerase chain reaction method.

As the PCR method, the aforementioned quantitative polymerase chain reaction method is preferred. That is, the composition of the present invention is preferably used in the quantitative polymerase chain reaction method.

The composition of the present invention can be prepared by dissolving the sensitizer of the present invention in a solvent such as water, together with, where necessary, other components used for nucleic acid amplification. That is, the composition of the invention is preferably a composition containing the sensitizer of the invention and water (and other components where necessary). The concentration of the sensitizer of the present invention in the composition of the present invention is as described above.

As other components used for nucleic acid amplification, known components used for known nucleic acid amplification represented by PCR can be used. Examples of such component include buffer, substrate, primer, DNA polymerase, fluorescent DNA staining reagent, fluorescent probe, passive reference, nucleic acid, and the like. Only one kind of other component may be used, or two or more kinds thereof may be used in combination.

Buffer is not particularly limited. Examples thereof include buffers with a pH adjusted to 6 to 9, more preferably about 7 to 8, by mixing a base such as tris(hydroxymethyl)aminomethane, Tricine, Bicine, and the like and an acid such as sulfuric acid, hydrochloric acid, acetic acid, phosphoric acid, and the like. The buffer desirably contains a magnesium salt and/or a manganese salt as appropriate. The buffer may further contain a salt such as potassium chloride, ammonium sulfate, and the like. The buffer may further contain a water-soluble organic solvent such as dimethyl sulfoxide, dimethylformamide, formamide, glycerol, and the like. The buffer may further contain a surfactant such as polyoxysorbitan fatty acid ester, polyoxyethylene alkylphenyl ether, and the like. The buffer may further contain a protein such as bovine serum albumin and the like.

While the substrate is not particularly limited, examples thereof include a mixture (dNTPs) of deoxyadenosine triphosphate (dATP), deoxythymidine triphosphate (dTTP), deoxyguanosine triphosphate (dGTP), and deoxythymidine triphosphate (dCTP). It is also possible to partially and/or entirely substitute dTTP with deoxyuridine triphosphate (dUTP). In sequencing PCR and the like, an appropriate amount of a mixture of dideoxyadenosine triphosphate (ddATP), dideoxythymidine triphosphate (ddTTP), dideoxyguanosine triphosphate (ddGTP), and dideoxythymidine triphosphate (ddCTP), or a fluorescently labeled product thereof may also be added.

Examples of the primer include oligonucleotides with a length of 10 to 40 bases. The length of aforementioned oligonucleotide is preferably 15 to 30 bases, more preferably 15 to 25 bases. The aforementioned oligonucleotide can be designed and prepared by known methods. The aforementioned oligonucleotides may have a group that emits fluorescence and formed from fluorescein or the like.

Only one kind of primer may be used, or two kinds thereof may be used in a pair. A plurality of primers may also be used to simultaneously amplify plural regions. The concentration of the primer in the composition of the present invention is preferably 0.1 to 25 $\mu$M, more preferably 0.1 to 15 $\mu$M, further preferably 0.5 to 10 $\mu$M.

As the DNA polymerase, known DNA polymerase can be used. From the aspect of heat resistance, enzymes derived from thermophilic bacteria, thermophilic archaea, hyperthermophile, or hyperthermophile archaea, and mutant enzymes thereof are preferred. A DNA polymerase is appropriately selected from a DNA-dependent DNA polymerase, an RNA-dependent DNA polymerase, or an enzyme having both functions, depending on the purpose of nucleic acid amplification. Moreover, whether to use a DNA polymerase having nuclease activity or a DNA polymerase having no nuclease activity is appropriately determined.

The fluorescent DNA staining reagent is not particularly limited, and examples thereof include SYBR™ Green I and the like.

The fluorescent probe is not particularly limited, and examples thereof include TaqMan™ probe.

Passive reference may be selected appropriately according to the purpose of nucleic acid amplification. Examples of the passive reference include ROX™ Dye and the like.

As the nucleic acid, any DNA and/or RNA may be used, for example, as an exogenous control gene, in addition to the aforementioned primers and fluorescent probes. Here, the nucleic acid may be synthesized in vitro, or may be prepared from cells, microorganisms, viruses, or the like by known methods. Here, the cells, microorganisms, viruses, and the like may be those collected from human, animals, or plants in the natural world or environment, or may be those obtained by isolation and culture.

The composition of the present invention may further contain oil such as mineral oil and the like; solid phase carrier such as glass beads, magnetic beads, and the like.

In addition, kit-type products in which a plurality of the above-mentioned components are combined, or master-mix (sometimes called primer mix, pre-mix, etc.) type products in which those components are mixed in advance may

also be used.

[Test kit]

The composition of the present invention can be made into a test kit by combining with necessary configurational members. The present invention provides a test kit containing the composition of the present invention. The aforementioned configurational members are not particularly limited, and examples thereof include instrument for sample collection, container for sample collection, reagent for sample pretreatment, standard product for calibration, instrument for test, consumable, measurement cassette, manual, and the like. The aforementioned configurational members are appropriately selected according to the mode of test. The aforementioned configurational members may be included in a test kit. In addition, commercially available products may be used as the aforementioned configurational members. As the aforementioned configurational members, those of specified standards may also be used.

The test kit of the present invention is used, for example, for genetic testing, microbial testing, and viral testing, preferably for viral testing. That is, the test object of the test reagent test kit of the present invention is preferably a virus.

Examples of the test in which the test kit of the present invention is used include tests performed in the fields of medicine, veterinary medicine, forensic medicine, pharmaceutical analysis, food analysis, environmental research, and the like. Among these, tests performed in the fields of medicine and veterinary medicine are preferred, and tests performed in the medical field are more preferred. The test kit of the present invention is preferably for clinical tests, more preferably ex-vivo diagnosis.

[Nucleic acid amplification method]

The present invention also provides (i) method for amplifying a nucleic acid, including using the polymer of the present invention as a sensitizer, and (ii) a method for amplifying a nucleic acid, including preparing a reaction liquid for nucleic acid amplification by mixing the composition of the present invention and a sample comprising a nucleic acid to be amplified. The polymer of the present invention and the composition of the present invention in the nucleic acid amplification method of the present invention are as described above. In addition, the nucleic acid amplification method is as described above unless particularly described.

The sample used in the nucleic acid amplification method of the present invention contains nucleic acids to be amplified. The sample is preferably a sample solution containing water and nucleic acids to be amplified. The sample may contain one kind of nucleic acid, or may contain two or more kinds of nucleic acid. The concentration of the nucleic acid in the sample is appropriately determined according to the purpose of nucleic acid amplification. When concentration preparation is possible, it is preferably 1 to $10^{20}$ copy/$\mu$L, more preferably 1 to $10^{10}$ copy/$\mu$L, further preferably 1 to $10^5$ copy/$\mu$L, particularly preferably 1 to 500 copy/$\mu$L, most preferably 1 to 100 copy/$\mu$L.

In the diffusion amplification method of the present invention, the amount of a sample used is preferably a trace amount to 1 $\mu$L, more preferably 0.01 to 1 $\mu$L, further preferably 0.2 to 0.4 $\mu$L, per 1 $\mu$L of the amount of the composition of the present invention used.

The nucleic acid amplification method of the present invention is preferably a reverse transcription polymerase chain reaction method. The nucleic acid amplification method of the present invention is also preferably a quantitative polymerase chain reaction method.

[Example]

The present invention is explained in more detail in the following by referring to Examples and the like, which are not to be construed as limitative.

[Synthesis of polymer (sensitizer for nucleic acid amplification)]

[Synthetic Example 1]

As monomer (1), 40.0 g of 2-methacryloyloxyethylphosphorylcholine (hereinafter sometimes to be abbreviated as "MPC") was weighed into a glass flask for polymerization, 60.0 g of purified water was added to dissolve monomer (1), and 0.31 g of azobisisobutyronitrile (hereinafter to be referred to as "AIBN") was added as a polymerization initiator to the obtained solution. After the inside of the reaction container was sufficiently replaced with nitrogen, polymerization was performed by heating at 70°C for 6 hr while stirring. The obtained reaction solution was ice-cooled and added dropwise to diethyl ether to precipitate a polymer. The precipitate was collected by filtration, washed with diethyl ether, and vacuum dried to give a homopolymer (hereinafter to be indicated as "polymer 1") as a white powder. The weight average molecular weight of polymer 1 was 1,030,000 based on polyethylene glycol as measured by gel filtration

chromatography (hereinafter sometimes to be abbreviated as "GPC") under the below-mentioned conditions.

[Synthetic Example 2]

MPC (6.0 g) as monomer (1) and methacrylic acid (hereinafter sometimes to be abbreviated as "MA") (4.0 g) as monomer (2) (monomer (1)/monomer (2)=30/70 (molar ratio)) were weighed into a glass flask for polymerization, 90.0 g of purified water was added to dissolve monomers (1) and (2), and AIBN (0.78 g) was added to the obtained solution. Thereafter, in the same manner as in Synthetic Example 1, a random copolymer (hereinafter to be indicated as "polymer 2") was obtained. The weight average molecular weight of polymer 2 was 680,000 based on polyethylene glycol as measured by GPC under the below-mentioned conditions.

[Synthetic Example 3]

MPC (19.4 g) as monomer (1) and butyl methacrylate (hereinafter sometimes to be abbreviated as "BMA") (2.2 g) as monomer (2) (monomer (1)/monomer (2)=80/20 (molar ratio)) were weighed into a glass flask for polymerization, 39.3 g of purified water and 39.3 g of ethanol were added to dissolve monomers (1) and (2), and AIBN (0.02 g) was added to the obtained solution. The inside of the flask was sufficiently replaced with nitrogen, and polymerization was performed by heating at 60°C for 5 hr while stirring. Thereafter, in the same manner as in Synthetic Example 1, a random copolymer (hereinafter to be indicated as "polymer 3") was obtained. The weight average molecular weight of polymer 3 was 600,000 based on polyethylene glycol as measured by GPC under the below-mentioned conditions.

[Synthetic Example 4]

MPC (11.7.0 g) as monomer (1) and stearyl methacrylate (hereinafter sometimes to be abbreviated as "SMA") (3.3 g) as monomer (2) (monomer (1)/monomer (2)=80/20 (molar ratio)) were weighed into a glass flask for polymerization, 85 g of ethanol were added to dissolve monomers (1) and (2), and AIBN (0.06 g) was added to the obtained solution. The inside of the flask was sufficiently replaced with nitrogen, and polymerization was performed by heating at 60°C for 6 hr while stirring. Thereafter, in the same manner as in Synthetic Example 1, a random copolymer (hereinafter to be indicated as "polymer 4") was obtained. The weight average molecular weight of polymer 4 was 43,000 based on polyethylene glycol as measured by GPC under the below-mentioned conditions.

[Synthetic Example 5]

MPC (8.4 g) as monomer (1), BMA (2.1 g) as monomer (2), and glycerol monomethacrylate (hereinafter sometimes to be abbreviated as "GLM") (4.5 g) as monomer (3) (monomer (1)/monomer (2)/monomer (3)=40/40/20 (molar ratio)) were weighed into a glass flask for polymerization, 42.5 g of purified water and 42.5 g of ethanol were added to dissolve monomers (1), (2), and (3), and AIBN (0.15 g) was added to the obtained solution. Thereafter, in the same manner as in Synthetic Example 3, a random copolymer (hereinafter to be indicated as "polymer 5") was obtained. The weight average molecular weight of polymer 5 was 22,000 based on polyethylene glycol as measured by GPC under the below-mentioned conditions.

[GPC measurement]

GPC measurement of the polymers 1 to 5 obtained in Synthetic Examples 1 to 5 was performed under the following conditions.

GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 μL

The monomers used in Synthetic Examples 1 to 5 and molar ratios thereof, and weight average molecular

weights of the obtained polymers are summarized in Table 1.

[Table 1]

| | monomer (1) | monomer (2) | monomer (3) | molar ratio of monomers | weight average molecular weight |
|---|---|---|---|---|---|
| polymer 1 | MPC | - | - | 100/0/0 | 1,030,000 |
| polymer 2 | MPC | MA | - | 30/70/0 | 680,000 |
| polymer 3 | MPC | BMA | - | 80/20/0 | 600,000 |
| polymer 4 | MPC | SMA | - | 80/20/0 | 43,000 |
| polymer 5 | MPC | BMA | GLM | 40/40/20 | 22,000 |
| molar ratio of monomers = monomer (1)/monomer (2)/monomer (3)<br>MPC: 2-methacryloyloxyethyl phosphorylcholine<br>MA: methacrylic acid<br>BMA: butyl methacrylate<br>SMA: stearyl methacrylate<br>GLM: glycerol monomethacrylate | | | | | |

[Examples 1 to 5 and Comparative Example 1]

[Sample solution]

Positive Control RNA, N set No.2 (N2) attached to the SARS-CoV-2 RT-qPCR Detection Kit (manufactured by FUJIFILM Wako Pure Chemical Corporation) was diluted with nuclease-free purified water (hereinafter sometimes to be indicated as "PW (nuclease-free)") to 10 copy/μL according to the package insert of the aforementioned kit to prepare a sample solution. PW (nuclease-free) was used as a negative control instead of the aforementioned sample solution.

[Reaction liquid]

Using the components of the aforementioned kit and 50x ROX Passive Reference (manufactured by NIPPON GENE CO., LTD.), solutions having a common composition shown in Table 2 were prepared, and predetermined amounts of the polymers obtained in Synthetic Examples 1 to 5 were added thereto. A predetermined amount of PW (nuclease-free) was added to prepare a composition for nucleic acid amplification. The obtained composition for nucleic acid amplification (15 μL) was dispensed onto a PCR plate (MicroAmp™ Fast Optical 96-well Reaction Plate with Barcode, 0.1 mL, manufactured by AppliedBioscience), and further spiked with 5 μL of the aforementioned sample solution to prepare a total 20 μL of the reaction liquid. The composition of the obtained reaction liquid is shown in the following Table 2.

In the above-mentioned operation, 2 μL of the polymer aqueous solution having a concentration that is 10 times the final concentration of the polymer in the reaction liquid was added. For example, "under the condition where the final concentration of polymer 1 in the reaction liquid is 0.1 w/v%", 1 w/v% aqueous solution (2 μL) of polymer 1 and a solution (13 μL) of components of the common composition and PW (nuclease-free) were mixed to prepare a composition for nucleic acid amplification (15 μL). Thereto was added the sample solution (5 μL) to prepare the reaction liquid (20 μL).

[Table 2]

<table>
<tr><td colspan="3"></td><td>reagent name</td><td>amount added</td></tr>
<tr><td rowspan="10">composition for nucleic acid amplification</td><td rowspan="8">common composition</td><td>5xReaction Buffer*</td><td>4 μL</td></tr>
<tr><td>2 mmol/L dNTPs*</td><td>4 μL</td></tr>
<tr><td>50 mmol/L Manganese (I I) Acetate *</td><td>1 μL</td></tr>
<tr><td>Hot Start Reverse Transcription DNA Polymerase*</td><td>0.25 μL</td></tr>
<tr><td>Fw Primer NIID_2019-nCOCN_F2 (10 μmol/L)*</td><td>1 μL</td></tr>
<tr><td>Rv Primer NIID_2019_nCOV_N_R2 (10 μmol/L)*</td><td>1.4 μL</td></tr>
<tr><td>TaqMan Probe NIID_2019-nCOV_ N_P2 (5 μmol/L)*</td><td>0.8 μL</td></tr>
<tr><td>50x ROX Passive Reference**</td><td>0.4 μL</td></tr>
<tr><td colspan="2">any aqueous solution of polymer obtained in Synthetic Examples 1 to 5</td><td>2 μL</td></tr>
<tr><td colspan="2">Distilled Water, Nuclease-free*</td><td>rest</td></tr>
<tr><td colspan="3">amount of composition for nucleic acid amplification</td><td>15 μL</td></tr>
<tr><td colspan="3">amount of sample solution</td><td>5 μL</td></tr>
<tr><td colspan="3">amount of reaction liquid (=composition for nucleic acid amplification + sample solution)</td><td>20 μL</td></tr>
<tr><td colspan="4">* component of SARS-CoV-2 RT-qPCR Detection Kit (manufactured by FUJIFILM Wako Pure Chemical Corporation)<br>** manufactured by NIPPON GENE CO., LTD.</td></tr>
</table>

[Reaction]

The PCR plate into which the aforementioned reaction liquid was dispensed was set in the StepOnePlus™ Real-Time PCR System (manufactured by AppliedBioscience) and RT-qPCR method was performed according to the temperature program shown in Table 3. The reading of the fluorescence intensity was performed in step #5 in Table 3.

[Table 3]

<table>
<tr><td>step #</td><td>temperature</td><td>time</td><td>cycle number</td></tr>
<tr><td>1</td><td>90°C</td><td>30 sec</td><td></td></tr>
<tr><td>2</td><td>60°C</td><td>5 min</td><td></td></tr>
<tr><td>3</td><td>95°C</td><td>1 min</td><td></td></tr>
<tr><td>4</td><td>95°C</td><td>3 sec</td><td rowspan="2">60 cycles</td></tr>
<tr><td>5</td><td>60°C</td><td>20 sec</td></tr>
<tr><td>6</td><td>4°C</td><td>∞</td><td></td></tr>
</table>

[Results]

Under each condition shown in Table 4-1, the RT-qPCR method was performed according to the above-mentioned procedure, and the endpoint enhancement rate was calculated from the endpoint fluorescence intensity and the following formula:

$$\text{endpoint enhancement rate (\%)} = 100\times(F-F_0)/F_0$$

wherein F is the endpoint fluorescence intensity when the polymer is added, and $F_0$ is the endpoint fluorescence intensity when the polymer is not added (Comparative Example 1)). As used herein, the endpoint fluorescence intensity refers to the fluorescence intensity in the final cycle (60th cycle) of the PCR method, and the fluorescence intensity refers to the ΔRn value. The results are shown in Table 4-1.

[Table 4-1]

| | Comparative Example 1 | Example 1 | Example 2 | Example 3 | Example 4 | Example 5 |
|---|---|---|---|---|---|---|
| kind of polymer used | - | polymer 1 | polymer 2 | polymer 3 | polymer 4 | polymer 5 |
| concentration of polymer in composition for nucleic acid amplification | - | 0.13 w/v% | 0.0067 w/v% | 0.067 w/v% | 0.067 w/v% | 0.067 w/v% |
| final concentration of polymer in reaction liquid | - | 0.1 w/v% | 0.005 w/v% | 0.05 w/v% | 0.05 w/v% | 0.05 w/v% |
| endpoint enhancement rate | - | 583 % | 372 % | 404 % | 454 % | 455 % |
| concentration of nucleic acid in sample solution: 10 copy/μL | | | | | | |

[Comparative Examples 2 to 5]

In Comparative Examples 2 to 5, the sensitizer of the present invention used in Examples 1 to 5 (i.e., polymers 1 to 5) was replaced with each additive shown in Table 4-2 (i.e., bovine serum albumin (hereinafter indicated as "BSA", manufactured by Sigma-Aldrich), dimethyl sulfoxide (hereinafter indicated as "DMSO"), polyethylene glycol 6000 (hereinafter indicated as "PEG 6000"), or T4 Gene 32 Protein (manufactured by NIPPON GENE CO., LTD.)), and endpoint enhancement rate (%) was calculated. Specifically, under each condition shown in Table 4-2, the RT-qPCR method was performed according to the procedures of the aforementioned [Examples 1 to 5 and Comparative Example 1], and the endpoint enhancement rate was calculated from the endpoint fluorescence intensity and the following formula:

$$\text{endpoint enhancement rate (\%)} = 100\times(F-F_0)/F_0$$

wherein F is the endpoint fluorescence intensity when the additive is added, and $F_0$ is the endpoint fluorescence intensity when the additive is not added (Comparative Example 1)). The results are shown in Table 4-2.

Table 4-2]

| | Comparative Example 2 | Comparative Example 3 | Comparative Example 4 | Comparative Example 5 |
|---|---|---|---|---|
| kind of additive used | BSA | DMSO | PEG 6000 | T4 Gene32 Protein |
| concentration of additive in composition for nucleic acid amplification | 0.13 w/v% | 2.67 vol% | 0.13 w/v% | 2.0 μM |
| final concentration of additive in reaction liquid | 0.1 w/v% | 2 vol% | 0.1 w/v% | 1.5 μM |
| endpoint enhancement rate | 128% | 102% | 157% | 7 6% |
| concentration of nucleic acid concentration in sample solution: 10 copy/μL | | | | |

As shown in Table 4-1, the endpoint enhancement rate in Examples 1 to 5 using the sensitizers of the present invention (i.e., polymers 1 to 5) was 372 to 583%. In contrast, as shown in Table 4-2, the endpoint enhancement rate

in Comparative Example 2 to 5 using additives (i.e., BSA, DMSO, PEG 6000, or T4 Gene 32 Protein) other than the sensitizer of the present invention was 76 to 157%. From these results, it is found that the detection sensitivity of the measurement target, nucleic acid, can be improved by the use of the sensitizer of the present invention.

[Examples 6 to 10 and Comparative Example 6]

[Sample]

Positive Control RNA, N set No.2 (N2) attached to the SARS-CoV-2 RT-qPCR Detection Kit (manufactured by FUJIFILM Wako Pure Chemical Corporation) was diluted with PW (nuclease-free) according to the package insert of the aforementioned kit to prepare a sample solution with a nucleic acid concentration of 160, 40, 10, 2.5, or 0.0625 copy/μL. PW (nuclease-free) was used as a negative control instead of the aforementioned sample solution.

[Reaction liquid]

Using the components of the aforementioned kit, 50x ROX Passive Reference (manufactured by NIPPON GENE CO., LTD.), and the polymers obtained in Synthetic Examples 1 to 5, and in the same manner as in Examples 1 to 5 and Comparative Example 1, composition for nucleic acid amplification (15 μL) was prepared. The composition for nucleic acid amplification was spiked with 5 μL of the aforementioned sample solution to prepare a total 20 μL of the reaction liquid. The final concentration of the polymer in the reaction liquid is as shown in the following Table 5.

[Reaction]

In the same manner as in Examples 1 to 5 and Comparative Example 1, RT-qPCR method was performed.

[Results]

Under each condition shown in Table 5, RT-qPCR method was performed according to the procedure described above. Reactions were performed with N=2 for each condition, and those in which amplification was observed in two were judged as "strongly detected" (indicated as "PP" in Table 5), those in which amplification was observed in one were judged as "weakly detected" (indicated as "P" in Table 5), and those in which amplification was not observed in two were judged as "no detection" (indicated as "N" in Table 5). In addition, the lowest nucleic acid concentration in the sample solutions judged to be "strongly detected" and "weakly detected" was judged to be the lower detection limit thereof. In the case of no judgment of "weakly detected", it was judged that "lower detection limit of weakly detected" = "lower detection limit of strongly detected". The results are shown in Table 5.

[Table 5]

| | Comparative Example 6 | Example 6 | Example 7 | Example 8 | Example 9 | Example 10 |
|---|---|---|---|---|---|---|
| polymer used | - | polymer 1 | polymer 2 | polymer 3 | polymer 4 | polymer 5 |
| concentration of polymer in composition for nucleic acid amplification | - | 0.13 w/v% | 0.0067 w/v% | 0.067 w/v% | 0.067 w/v% | 0.067 w/v% |
| final concentration of polymer in reaction liquid | - | 0.1 w/v% | 0.005 w/v% | 0.05 w/v% | 0.05 w/v% | 0.05 w/v% |

(continued)

| | | Comparative Example 6 | Example 6 | Example 7 | Example 8 | Example 9 | Example 10 |
|---|---|---|---|---|---|---|---|
| concentration of nucleic acid in sample solution | 160 copy/μL | PP | PP | PP | PP | PP | PP |
| | 40 copy/μL | PP | PP | PP | PP | PP | PP |
| | 10 copy/μL | P | P | PP | PP | PP | PP |
| | 2.5 copy/μL | N | P | N | N | P | P |
| | 0.625 copy/pL | N | N | N | N | P | N |
| detection lower limit | strongly detected | 40 copy/μL | 40 copy/μL | 10 copy/μL | 10 copy/pL | 10 copy/μL | 10 copy/μL |
| | weakly detected | 10 copy/μL | 2.5 copy/pL | 10 copy/μL | 10 copy/μL | 0.625 copy/μL | 2.5 copy/pL |

As shown in Table 5, the lower detection limit of "strongly detected" and/or "weakly detected" decreased in Examples 6 to 10 using the sensitizers of the present invention (i.e., polymers 1 to 5) as compared with Comparative Example 6 without using the sensitizers. From these results, it is found that the detection sensitivity of the measurement target, nucleic acid, can be improved by the use of the sensitizer of the present invention.

[Industrial Applicability]

Using the sensitizer of the present invention, the detection sensitivity of nucleic acid as a measurement target in the nucleic acid amplification method (particularly, PCR method) can be improved. The sensitizer of the present invention can be preferably used in nucleic acid amplification methods for genetic testing, microbial testing, viral testing, and the like.

This application is based on a patent application No. 2021-12400 filed in Japan, the contents of which are incorporated in full herein.

## Claims

1. A sensitizer for nucleic acid amplification which is a polymer comprising a constitutional unit derived from a monomer represented by the formula (1):

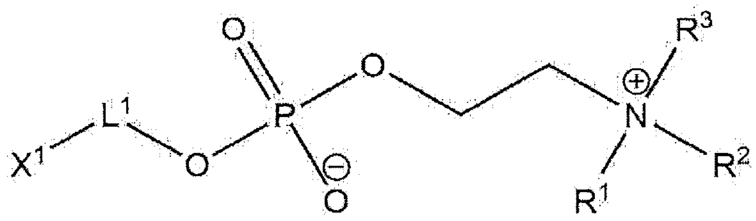

(1)

wherein

$X^1$ is a (meth)acryloyloxy group or a (meth)acryloylamino group,
$L^1$ is an alkylene group having 2 to 4 carbon atoms and optionally having one hydroxy group, or an alkyleneoxyalkylene group having 2 to 4 carbon atoms, and
$R^1$ to $R^3$ are each independently an alkyl group having 1 to 3 carbon atoms.

2. The sensitizer according to claim 1, wherein the sensitizer is a homopolymer comprising one kind of constitutional unit derived from the monomer represented by the formula (1).

3. The sensitizer according to claim 1, wherein the sensitizer is a copolymer further comprising a constitutional unit derived from a monomer represented by the formula (2):

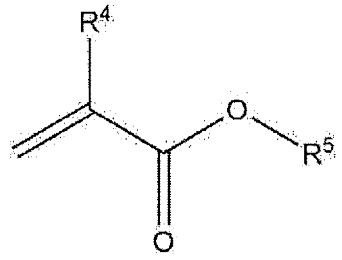

(2)

wherein

$R^4$ is a hydrogen atom or a methyl group, and
$R^5$ is a hydrogen atom or an alkyl group having 1 to 20 carbon atoms.

4. The sensitizer according to claim 3, wherein $R^5$ is an alkyl group having 12 to 18 carbon atoms.

5. The sensitizer according to claim 1, 3, or 4, wherein the sensitizer is a copolymer further comprising a constitutional unit derived from a monomer represented by the formula (3):

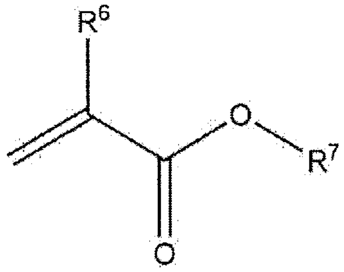

(3)

wherein

$R^6$ is a hydrogen atom or a methyl group, and
$R^7$ is an alkyl group having 3 to 6 carbon atoms, and two or more hydroxy groups.

6. A composition for nucleic acid amplification, comprising the sensitizer according to any one of claims 1 to 5.

7. The composition according to claim 6, wherein the composition is used for a reverse transcription polymerase chain reaction method.

8. The composition according to claim 6 or 7, wherein the composition is used for a quantitative polymerase chain reaction method.

9. A test kit comprising the composition according to any one of claims 6 to 8.

10. The test kit according to claim 9, wherein the test kit is for a clinical test.

11. The test kit according to claim 9 or 10, wherein an object of the test is a virus.

## INTERNATIONAL SEARCH REPORT

International application No.
**PCT/JP2022/002102**

**A. CLASSIFICATION OF SUBJECT MATTER**

***C08F 120/34***(2006.01)i; ***C09K 3/00***(2006.01)i; ***C12Q 1/6844***(2018.01)i; ***C12Q 1/686***(2018.01)i; ***C12Q 1/6888***(2018.01)i
FI: C09K3/00 Z; C08F120/34; C12Q1/6844 Z; C12Q1/686 Z; C12Q1/6888 Z

According to International Patent Classification (IPC) or to both national classification and IPC

**B. FIELDS SEARCHED**

Minimum documentation searched (classification system followed by classification symbols)

C08F120/34; C09K3/00; C12Q1/6844; C12Q1/686; C12Q1/6888

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | US 2019/0360021 A1 (NANOHELIX CO., LTD.) 28 November 2019 (2019-11-28) claims 1, 11-13, paragraphs [0032]-[0037], [0060]-[0068], fig. 2-4, experiment examples 1-3 | 1, 3-4, 6-11 |
| A | | 2, 5 |
| A | JP 2005-87109 A (SUMITOMO BAKELITE CO LTD) 07 April 2005 (2005-04-07) entire text | 1-11 |
| A | JP 2013-223430 A (SUMITOMO BAKELITE CO LTD) 31 October 2013 (2013-10-31) entire text | 1-11 |
| A | JP 2006-230335 A (SUMITOMO BAKELITE CO LTD) 07 September 2006 (2006-09-07) entire text | 1-11 |
| A | JP 2007-155623 A (SEKISUI CHEMICAL CO LTD) 21 June 2007 (2007-06-21) entire text | 1-11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 March 2022** | **05 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/002102**

| Patent document cited in search report | Publication date (day/month/year) | Patent family member(s) | Publication date (day/month/year) |
|---|---|---|---|
| US 2019/0360021 A1 | 28 November 2019 | WO 2019/103164 A1<br>entire text<br>KR 10-2019-0088559 A | |
| JP 2005-87109 A | 07 April 2005 | (Family: none) | |
| JP 2013-223430 A | 31 October 2013 | (Family: none) | |
| JP 2006-230335 A | 07 September 2006 | (Family: none) | |
| JP 2007-155623 A | 21 June 2007 | CN 101341408 A<br>entire text | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007004654 A
- WO 2018216628 A